# EUROPÄISCHE PATENTSCHRIFT

(11) **EP 0 873 724 B1**
(45) Veröffentlichungstag und Bekanntmachung des Hinweises auf die Patenterteilung: **16.07.2003**
(21) Anmeldenummer: 98710008.8
(22) Anmeldetag: 17.04.1998
(51) Int. Cl.: A61C 1/08

(54) **Handstück für medizinische Zwecke, insbesondere für eine ärztliche oder zahnärztliche Behandlungseinrichtung, vorzugsweise für eine spanabhebende Bearbeitung eines Zahn-Wurzelkanals**
Handpiece for medical purposes, especially for a medical or dental treatment apparatus, preferably for use in root canal procedures
Pièce à main pour applications médicales, en particulier pour dispositif de traitement medical ou dentaire, de préférence pour la préparation de canaux dentaires

(30) Priorität: 18.04.1997 DE 19716416; 13.10.1997 DE 19745245
(43) Veröffentlichungstag der Anmeldung: 28.10.1998
(73) Patentinhaber: Kaltenbach & Voigt GmbH & Co. KG, 88400 Biberach/Riss (DE)
(72) Erfinder: Logé, Hans, 88400 Biberach (DE); Gugel, Bernd, 89079 Ulm-Einsingen (DE); Kuhn, Bernhard, 88433 Schemmerhofen (DE)
(74) Vertreter: Schmidt-Evers, Jürgen, Dipl.-Ing.

(56) Entgegenhaltungen:
- DE-A- 3 207 489
- FR-A- 2 544 812
- US-A- 3 578 745
- US-A- 4 338 798

## Beschreibung

Die Erfindung bezieht sich auf ein Handstück nach dem Oberbegriff des Anspruchs 1 oder 2 oder 3.

Bei einem solchen Handstück kann es sich unter anderem um ein ärztliches oder zahnärztliches Behandlungsinstrument oder um ein Bearbeitungsinstrument für ein medizinisches oder zahntechnisches Labor handeln.

Ärztliche oder zahnärztliche Handstücke unterscheiden sich unter anderem durch die Funktion des von ihnen jeweils getragenen Werkzeugs und die Punktion des Antriebs. Ein Werkzeug kann durch Rotation, wie es z. B. bei einem Bohrer der Fall ist, oder durch eine axiale Hubbewegung, wie es bei einer Feile der Fall ist, oder auch in Kombination dieser Bewegungen, angetrieben sein. Ein Handstück mit einem rotierend angetriebenen Werkzeug kann bei entsprechender Ausbildung des Werkzeugs auch dazu benutzt werden, eine Drehantriebskraft auf Drehteile zu übertragen, z. B. am Behandlungsort vorhandene Schrauben anzuziehen oder zu lösen, wie es z. B. bei Schrauben von Zahnersatz oder schraubbaren Implantaten oder sonstigen schraubbaren Körperteilen der Fall ist.

Die Benutzung eines vorliegenden Handstücks ist aus mehreren Gründen problematisch. Ein Problem besteht darin, daß aufgrund der stabförmigen Ausbildung des Handstücks mit ihm eine beträchtliche Hebelwirkung bei einer Schwenkung um die Werkzeugsachse ausgeübt werden kann, die hinsichtlich einer Belastung sowohl auf das Werkzeug als auch auf den Zahn zu Überbeanspruchungen führen kann, z. B. zum Abbrechen des Werkzeugs oder zu einer Aushebelungsbeanspruchung des Zahns. Das vorliegende Problem stellt sich insbesondere dann, wenn es sich bei dem Werkzeug um ein solches handelt, das zur Aufbereitung einer Kavität dient, in der das Werkzeug abbrechen kann, vor allem dann, wenn die Querschnittsgröße des Werkzeugs verhältnismäßig klein ist, wie es bei einem Behandlungs- und Bearbeitungswerkzeug zur Aufbereitung eines Wurzelkanals eines Zahns der Fall ist. Wenn ein solches Werkzeug bei der Behandlung bricht, sitzt es verhältnismäßig fest in dem ihn aufnehmenden Hohlraum und es kann in den meisten Fällen nicht entfernt werden. Es ist damit zu rechnen, daß ein abgebrochenes Wurzelkanalwerkzeug die Funktion des Zahns und dessen Lebensdauer beeinträchtigt oder einen Entzündungsherd einleiten kann, der zum vorzeitigen Verlust des Zahns führt.

Die vorbeschriebenen Probleme stellen sich insbesondere bei einem Handstück mit einem Wurzelkanalwerkzeug, weil ein solches Werkzeug aufgrund seiner verhältnismäßig kleinen Querschnittsgröße und Länge leicht bricht und ein Bruchstück im Wurzelkanal verklemmt und in den meisten Fällen nicht herausgeholt werden kann.

Bei einem solchen Handstück, bei dem das Handstück bzw. das Werkzeug eine Drehantriebsfunktion erfüllt können ebenfalls aus einer Überlastung resultierende Probleme auftreten, z. B. dann, wenn eine Gewindeschraube, die zur Befestigung eines Zahns oder eines Implantats oder eines Körperteils dient oder ein Implantat bildet, überdreht wird.

In der DE-A-320 7489 ist ein zahnärztliches Handstück nach dem Oberbegriff des Anspruchs 1 oder 2 oder 3 beschrieben, beide aus dem Antrieb resultierende Überbelastungen des Werkzeugs oder des zu behandelnden Gegenstandes vermieden sind.

Bei dieser vorbekannten Ausgestaltung ist in die Antriebsverbindung des Werkzeugs eine Überlastkupplung integriert, die auf einen maximalen Antriebs- oder Drehmoment-Übertragungswert eingestellt ist und deshalb selbsttätig löst, wenn das vorbestimmte Antriebsmoment überschritten wird. Hierdurch wird eine Überlastung des Werkzeugs selbst oder eines mit dem Werkzeug beanspruchtes Körper- oder Körperersatzteils z.B. eines Implantats, vermieden. Dabei bilden das Handstück und das Werkzeug eine Antriebs-, insbesondere eine Drehantriebsvorrichtung.

Der Erfindung liegt die Aufgabe zugrunde, ein Handstück nach dem Oberbegriff des Anspruchs 1 oder 2 oder 3 so auszugestalten, daß es sich bei Vermeidung von Überbelastungen für eine Benutzung mit unterschiedlichen Drehmomentwerten eignet. Im weiteren soll bei einem insbesondere für eine Wurzelkanalbehandlung oder für einen Schraubantrieb eingerichteten Handstück die Handhabbarkeit verbessert werden.

Diese Aufgabe wird durch die Merkmale des Anspruchs 1 oder 2 oder 3 gelöst.

Bei den erfindungsgemäßen Ausgestaltungen ist der wirksame Drehmomentwert in seiner Größe einstellbar. Dabei ist ein von außen manuell zugängliches Einstellelement vorzusehen, das in wenigstens zwei Stellungen verschiebbar ist und dabei eine stufenlose Einstellung oder eine Einstellung in Stufen unterschiedlicher Drehmomentwerte bewirkt. Als Einstellelement eignet sich sehr vorteilhaft ein dünner Ring, der aus ergonomischen Gründen die Handhabung des Handstücks nicht beeinträchtigt.

Die Behandlung des menschlichen oder tierischen Körpers oder auch medizinischer Modelle oder künstlicher Körperteile erfordert in vielen Fällen eine Behandlung oder Bearbeitung in ergonomisch schwierigen Stellungen, wie es z. B. bei der Behandlung eines Zahns oder Zahnteiles im Oberkieferbereich oder auch im hinteren Mundraumbereich der Fall ist. Außerdem sind mit einem Handstück Präzisionsarbeiten auszuführen, wie es z. B. bei einer Wurzelkanalbehandlung der Fall ist, bei der sowohl aus Gründen der Präzision als auch aus Gründen der Bruchgefahr eine besonders sorgfältige Handhabung erforderlich ist. Übliche Handstücke sind zum Zweck ihrer Versorgung mit Antriebsenergie und auch mit besonderen Behandlungsmedien durch einen sogenannten flexiblen Versorgungsschlauch mit einer Versorgungseinrichtung verbunden. Obwohl der Versorgungsschlauch flexibel ist, führt er zu einer gewissen Beeinträchtigung der Handhabbarkeit des Handstücks.

Bei den erfindungsgemäßen Ausgestaltungen nach den Ansprüchen 2 oder 3 ist das Handstück unabhängig von einem Versorgungsschlauch, und es entfällt deshalb eine durch einen Versorgungsschlauch vorgegebene Beeinträchtigung der Bewegung des Handstücks. Ein weiterer Vorteil dieser erfindungsgemäßen Ausgestaltung besteht auch darin, daß das Handstück aufgrund seiner Unabhängigkeit von einer Versorgungseinrichtung der üblichen Bauweise als sogenanntes Taschengerät mitgenommen und auch an solchen Orten angesetzt werden kann, an denen eine übliche Versorgungseinrichtung nicht vorhanden ist.

Die in den Handstücken nach Anspruch 1 oder 3 enthaltene Überlastkupplung kann an verschiedenen Stellen der Antriebsverbindung vorgesehen sein. Es ist z. B. möglich, die Überlastkupplung in den Antriebswellenabschnitt oder in die Haltekupplung für das Werkzeug zu integrieren. Wesentlich ist, daß die Überlastkupplung als schwächstes Glied der Antriebsverbindung funktioniert und die angestrebte Sicherheitsfunktion ausübt.

Die Anordnungsstelle der Überlastkupplung kann insbesondere bei einem Handstück für eine zahnärztliche Behandlungseinrichtung wesentlich sein, da die Anordnung der Überlastkupplung zwangsläufig zu einer partiell größeren Bauweise führt und am Arbeitsort eines solchen Handstücks, nämlich im Mundraum des Patienten, zum einen wenig Raum vorhanden ist und zum anderen eine Vergrößerung der Bauweise den Blick auf die Behandlungsstelle erschwert, der für eine sorgfältige Beobachtung bei der Behandlung erforderlich ist.

Bei der erfindungsgemäßen Überlastkupplung ist die Drehmitnahme unterhalb des vorbestimmten Drehmomentwertes gewährleistet. Es eignet sich sehr vorteilhaft eine solche Kupplung, insbesondere eine Rutschkupplung, die oberhalb des vorbeschriebenen Drehmomentwertes durchrutscht und bei einer Verringerung des zu übertragenden Drehmomentes selbsttätig wieder in die Kupplungsfunktion gelangt. Diesen Zweck erfüllen sehr vorteilhaft zwei ebene und rechtwinklig zur vorhandenen Drehachse angeordnete Reibflächen oder zwei bezüglich der Kupplungsachse z.B. rotationssymmetrische Reibflächen an zwei Kupplungsscheiben, von denen eine oder beide axial verschiebbar gelagert ist bzw. sind und die durch eine Federkraft gegeneinander vorgespannt ist bzw. sind.

Im Rahmen der Erfindung ist es auch vorteilhaft, die einander zugewandten Kupplungsflächen der Kupplungsteile mit zueinander passenden, von einer gedachten Rotationsfläche quer abweichenden wellen- oder keilförmigen Erhebungen und Vertiefungen mit gleichen verhältnismäßig großen Flankenwinkeln von etwa 135° und mehr auszubilden, wobei das axiale Verschiebungsmaß einer oder beider Scheiben wenigstens dem axialen Maß der Erhebungen entspricht, um das Lösen der Überlastkupplung zu gewährleisten.

Im Rahmen der Erfindung ist es dabei möglich und vorteilhaft, wenn zwischen den Reibflächen der Kupplungsteile Wälzkörper, insbesonder Kugeln, im Sinne eines Kranzes angeordnet werden. Im weiteren ist es auch möglich, die Erhebungen und Vertiefungen durch Kalotten zu bilden, deren Querschnittsform vorzugsweise an die Querschnittsform der Wälzkörper angepaßt ist.

Bei einer Ausgestaltung mit den vorbeschriebenen zahnförmigen Eingriffsflächen ergeben sich ein wirksames Übertragungsmoment und beim Durchrutschen Vibrationen und kleine, in Umfangsrichtung wirksame Schläge, die die Wirksamkeit weiter verbessern und je nach Drehrichtung ein Lösen eines festsitzenden Werkzeugs oder eines damit beaufschlagten Teiles, wie eine Schraube, bewirken können.

Es ist im weiteren insbesondere für ein zahnärztliches Handstück vorteilhaft, die Überlastkupplung im hinteren Bereich des Handstücks anzuordnen, in dem eine durch die Überlastkupplung hervorgerufene partielle Vergrößerung der Bauweise weniger oder kaum stört, und wobei im Bereich des Handstückkopfes eine kleine Bauweise verwirklicht werden kann. Bei einem zahnärztlichen winkelförmigen Handstück empfiehlt es sich , die Überlastkupplung im hinteren Schenkel der Winkelform anzuordnen.

Um sowohl für eine Wurzelkanalbehandlung als auch eine andere Behandlung oder Bearbeitung, insbesondere für ein Drehwerkzeug, eine kleine Arbeitsgeschwindigkeit zu erreichen, ist es vorteilhaft, ein Reduziergetriebe in die Antriebsverbindung des Handstücks und somit auch in das Handstück zu integrieren.

Die erfindungsgemäße Ausgestaltung eignet sich gut für ein Handstück, das durch eine Schnellschlußverbindung wie eine Steckverbindung mit einem sogenannten Anschlußteil handhabungsfreundlich verbindbar und wieder lösbar ist, das durch einen sogenannten Versorgungsschlauch mit einer Steuereinrichtung einer Behandlungseinheit, z.B. einem Behandlungsstuhl verbunden ist.

Insbesondere dann, wenn das erfindungsgemäße Handstück mit der Überlastkupplung und einem Reduziergetriebe als Baueinheit ausgebildet ist, ist es möglich, ein für viele Behandlungs- oder Bearbeitungsarten ausgebildetes Anschlußteil auch als Antriebsteil für ein erfindungsgemäßes Handstück zu benutzen. Es bedarf somit für eine Vielzahl von unterschiedlichen Behandlungs- oder Bearbeitungsvorgängen nur eines gemeinsamen Anschlußteils, an das unterschiedliche Handstücke und auch das erfindungsgemäße Handstück wahlweise anmontierbar sind.

Nachfolgend werden die Erfindung und weitere durch sie erzielbare Vorteile anhand von Zeichnungen und vorteilhaften Ausgestaltungen näher beschrieben. Es zeigt
- Fig. 1: ein erfindungsgemäßes zahnärztliches Behandlungsinstrument mit einem Handstück im Längsschnitt;
- Fig. 2: ein erfindungsgemäßes Handstück in abgewandelter Ausgestaltung im Längsschnitt;
- Fig. 3: den Teilschnitt III-III in Fig. 2 in vergrößerter Darstellung;
- Fig. 4: ein Einzelteil des Behandlungsinstrumentes aus Fig. 2 in der Draufsicht;
- Fig. 5 bis 8: abgewandelte Ausgestaltungen des erfindungsgemäßen Handstücks nach Fig. 2 in vergrößerter Darstellung;
- Fig. 9: die in Fig. 2 mit X gekennzeichnete Einzeilheit in abgewandelter Ausgestaltung;
- Fig. 10: ein Teil der Ausgestaltung nach Fig. 9 in der Draufsicht;
- Fig. 11: die Einzelheit nach Fig. 9 in weiter abgewandelter Ausgestaltung;
- Fig. 12: ein Teil der Ausgestaltung nach Fig. 11 in der Draufsicht;
- Fig. 13: ein erfindungsgemäßes Handstück in abgewandelter Ausgestaltung in der Seitenansicht.

Die Hauptteile des allgemein mit 1 bezeichneten Behandlungsinstruments sind ein das hintere Ende des Behandlungsinstruments bildendes Anschlußteil 2 das Handstück 3, das durch eine Schnellkupplung 4, in Form einer Steckkupplung mit dem Anschlußteil 2 lösbar verbunden ist und im zusammengekuppelten Zustand sich in Form einer Griffhülse vom Anschlußteil 2 stabförmig nach vorne erstreckt, ein am vorderen Ende des Handstücks 3 angeordneter Werkzeughalter 5 für ein Behandlungs- oder Bearbeitungs- oder Drehwerkzeug 6, insbesondere ein Wurzelkanalwerkzeug, ein Antriebsmotor, vorzugsweise ein elektrischer Antriebsmotor 7, insbesondere im Anschlußteil 2, ein allgemein mit 8 bezeichneter Antriebswellenzug, der sich längs durch das eine Griffhülse bildende Handstück 3 erstreckt, und der aus mehreren Antriebswellenabschnitten 9, 10, 11, 12 besteht, die jeweils durch eine Kupplung miteinander verbunden oder verbindbar sind, ein dem Antriebswellenzug 8 im Bereich des Anschlußteils 2 oder des Handstücks 3 zugeordnetes Untersetzungsgetriebe 13, eine dem Antriebswellenzug 8 im Bereich des Anschlußteils 2 oder des Handstücks 3 zugeordnete Überlastkupplung 14, die beim Erreichen eines bestimmten Drehmomentwertes selbsttätig löst, und eine Einstellvorrichtung 15, mit der dieser Drehmomentwert veränderlich ist und somit die Überlastkupplung 14 einstellbar ist.

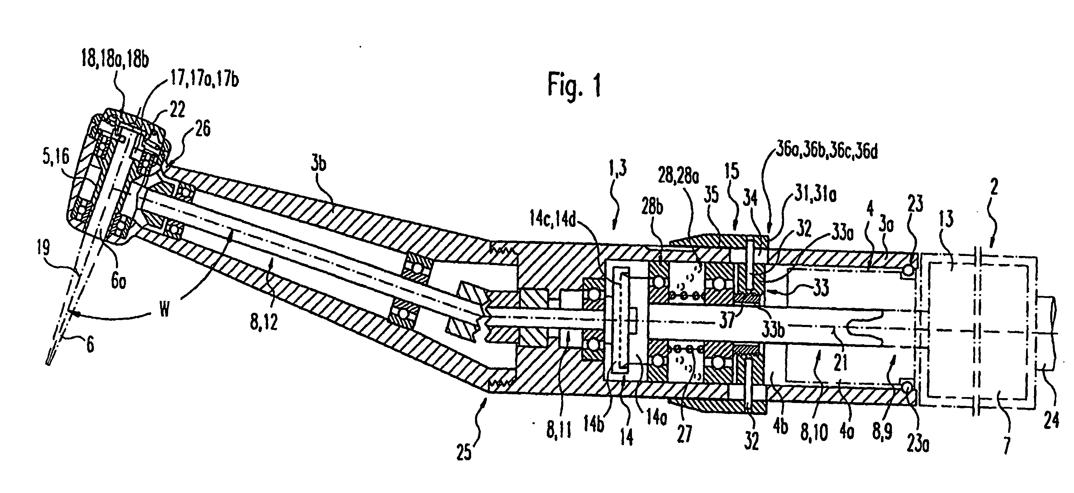

Bei der in Fig. 1 dargestellten Ausgestaltung erstreckt sich das Handstück 3 im Sinne eines stumpfen Winkels winkelförmig mit einem hinteren Handstückabschnitt 3a und einem vorderen Handstückabschnitt 3b. Das Handstück 3 kann sich jedoch auch gemäß einem noch zu beschreibenden weiteren Ausführungsbeispiel gerade erstrecken.

Der Werkzeughalter 5 ist beim vorliegenden Ausfuhrungsbeispiel durch eine Aufnahmehülse 16 gebildet, in die das Werkzeug 6 mit seinem Werkzeugschaft 6a einsteckbar und durch eine Drehmitnahmekupplung 17 und/oder eine axial wirksame Haltekupplung 18 mit der Aufnahmehülse 16 lösbar verbunden ist. Die Mittelachse 19 der Aufnahmehülse 16 und auch des Werkzeugs 6 ist quer zur Längsmittelachse 21 des Handstücks 3 bzw. des Behandlungsinstruments 1 gerichtet, wobei zwischen diesen Mittelachsen 19, 21 auf der Seite des Werkzeugs 6 ein stumpfer Winkel W von etwa 90° bis etwa 100° oder ein Winkel W von etwa 90° oder 100° eingeschlossen ist.

Die Drehmitnahmekupplung 17 weist ein Kupplungselement 17a auf, das in eine zum freien Ende des Werkzeugschafts 6a hin offene unrunde Kupplungsausnehmung 17b einfaßt, daß hier durch eine Abflachung des Werkzeugschaftes 6a gebildet ist. Die Haltekupplung 18 weist ein bezüglich der Mittelachse 19 radial verstellbar gelagertes Kupplungselement 18a auf, das durch eine Federkraft beim Einschieben des Werkzeugschaftes 6a in die Aufnahmehülse 16 selbsttätig in eine vorzugsweise ringförmige Kupplungsausnehmung 18b im Werkzeugschaft 6a einfaßt. Zum Lösen der Haltekupplung 18 ist auf der dem Werkzeug 6 abgewandten Seite des hier kopffömig verdickten Handstückendes ein Betätigungsglied 22 angeordnet, das bier vorzugsweise koaxial verschiebbar gelagert ist und bei einer mit Fingerdruck erzeugten Bewegung das Kupplungselement 18a in seine den Werkzeugschaft 6a freigebende Entkupplungsstellung verschiebt. Die Drehmitnahmekupplung 17 und die Haltekupplung 18 sind an sich bekannte Kupplungen, so daß eine detailierte Beschreibung entfallen kann.

Die Schnellkupplung 4 ist vorzugsweise durch eine Steckkupplung gebildet, die in der zusammengesteckten Stellung lösbar verrastet. Vorzugsweise ist diese Steckkupplung so ausgebildet, daß in der zusammengesteckten Stellung das Antriebsteil 2 und das Handstück 3 um die Längsmittelachse 21 relativ zueinander frei drehbar gelagert sind. Hierdurch wird die Handhabbarkeit des Handstücks 3 wesentlich verbessert, weil das Anschlußteil 2 an Drehbewegungen des Handstücks 3 während der Behandlung nicht teilzunehmen braucht. Die Dreh-Steckkupplung weist einen hohlzylindrischen Kupplungszapfen 4a am einen Kupplungsteil und eine diesen mit geringem Bewegungsspiel aufnehmende Kupplungsausnehmung 4b auf. Bei der vorliegenden Ausgestaltung erstreckt sich der Kupplungszapfen 4a vom Anschlußteil 2 nach vorne und die Kupplungsausnehmung 4b ist im hinteren Endbereich des Handstücks 3 angeordnet. Zur Verrastung in der Kupplungsstellung dient eine überdrückbare Verrastungsvorrichtung 23 mit einem Verrastungselement 23a, das in einer Ausnehmung in der Außenmantelfläche des Steckzapfens 4a oder in der Innenmantelfläche der Steckausnehmung 4b angeordnet ist und durch eine Feder in eine ihm jeweils gegenüberliegend im anderen Teil abgeordnete Verrastungsausnehmung so einfaßt, daß die Verrastungsvorrichtung 23 durch eine manuell leicht aufzubringende axiale Zugkraft überdrückbar ist.

Das Anschlußteil 2 ist durch einen andeutungsweise dargestellten flexiblen Versorgungsschlauch 24 mit einem nicht dargestellten Steuergerät verbunden, wie es bei einem zahnärztlichen Behandlungsplatz üblich ist. Durch die Versorgungsleitung 24 erstreckt sich eine Strom-Versorgungsleitung für den Antriebsmotor 7 und gegebenenfalls Medienleitungen mit Leitungen für Luft, Wasser und/oder Spray, die sich durch die Schnellkupplung 4 in an sich bekannter Weise in jeder Drehstellung funktionsfähig durchsetzend bis zum vorderen Endbereich des Handstücks 3 erstrecken, wo sie ausmünden und auf die Behandlungsstelle gerichtet sind.

Der erste Antriebswellenabschnitt 9 erstreckt sich vom Antriebsmotor 7 bis in den Bereich der Schnellkupplung 4, wo er mit dem zweiten Antriebswellenabschnitt 10 durch formschlüssig ineinander greifende Steckkupplungselemente verbunden ist, die beim Zusammenstecken der Schnellkupplung 4 gleichzeitig und selbsttätig gekuppelt werden. Der zweite Antriebswellenabschnitt 10 erstreckt sich nach vorne bis zur Überlastkupplung 14, die sich im mittleren Bereich des Handstücks 3, hier des hinteren Handstückschenkels 3a befindet. Von der Überlastkupplung 14 erstreckt sich der dritte Antriebswellenabschnitt 11 bis zum Scheitel 25 der Winkelform, in dessen Bereich ein Kegelradgetriebe angeordnet ist, dessen Zahnräder mit dem dritten und vierten Antriebswellenabschnitt 11, 12 drehfest verbunden sind. Der sich im vorderen Handstückabschnitt 3b erstreckende vierte Antriebswellenabschnitt 12 ist durch ein Zahnradgetriebe 26 oder Kegelradgetriebe mit der Aufnahmehülse 16 verbunden, wodurch deren Drehantrieb gewährleistet ist. Es ist auch möglich, hier ein solches Getriebe vorzusehen, daß die Drehbewegung des Antriebswellenabschnitts 12 in eine axiale Bewegung der Aufnahmehülse 16 umwandelt, wodurch ein axialer Hubantrieb für das Werkzeug 6 geschaffen ist. Es ist im weiteren auch möglich, das Getriebe zwischen dem Antriebswellenabschnitt 12 und der Aufnahmehülse 16 so auszubilden, daß es die Aufnahmehülse 16 und somit auch das Werkzeug 6 im Sinne einer axialen Hubbewegung und einer Drehbewegung antreibt.

Die Antriebswellenabschnitte 9 bis 12 sind jeweils durch geeignete Lager drehbar gelagert, vorzugsweise übliche Wälz- bzw. Kugellager.

Die Überlastkupplung 14 wird durch zwei Kupplungsbuchsen bzw -scheiben 14a, 14b gebildet, von denen die hintere Kupplungsscheibe 14a an ihrer Vorderseite und die vordere Kupplungsscheibe 14b an ihrer Rückseite jeweils eine Kupplungs- bzw. Reibfläche 14c, 14d aufweist, mit denen sie in einer rechtwinklig zur Längsmittelachse 21 angeordneten Drehebene aneinander liegen, wobei eine Kupplungsscheibe, hier die hintere Kupplungsscheibe 14a, axial verschiebbar gelagert und durch eine Feder 27, hier eine zylindrische oder kegelförmige Wendelfeder, gegen die andere Kupplungsscheibe 14b vorgespannt ist. Die Feder 27 ist mittelbar oder unmittelbar an der Griffhülse des Handstücks 3 abgestützt. Die Kupplungsscheiben 14a, 14b sind drehfest mit den zugehörigen Antriebswellenabschnitten 10, 11 verbunden. Eine der beiden Kupplungsscheiben 14a, 14b weist eine harte Reibfläche 14c auf, so daß nur die eine Kupplungsscheibe, hier die hintere Kupplungsscheibe 14a, ein Verschleißteil ist, daß für einen Ersatz ausgetauscht wird. Vorzugsweise handelt es sich hierbei um die Kupplungsscheibe 14a, die geringfügig axial verschiebbar gelagert ist und durch die Feder 27 in ihre Kupplungsstellung beaufschlagt ist.

Mit der Einstellvorrichtung 15 läßt sich die Druckspannung, mit der die Kupplungsscheiben 14a, 14b gegeneinander vorgespannt sind, verändern und folglich so einstellen, daß die Überlastkupplung 14 bei unterschiedlichen Drehmomentwerten öffnet, hier durchrutscht. Bei der vorliegenden Ausgestaltung wird mit der Einstellvorrichtung 15 die Vorspannung der Feder 27 verändert. Dies geschieht dadurch, daß das Widerlager 28 der Feder 27 axial verstellbar und in der jeweiligen Verstellposition feststellbar ist. Hierzu dient ein von außen manuell betätigbares Einstellglied 31, das mit einem Verbindungsglied 32 das Handstück 3 bzw. die Griffhülse radial nach innen durchsetzt und mit einem im Handstück 3 angeordneten Schieber 33 verbunden ist, der bei einer axialen Verschiebung des Einstellgliedes 31 nach vorn das Widerlager 28 ebenfalls vorschiebt und dabei die Vorspannung der Feder 27 vergrößert. Bei einer Verschiebung des Einstellgliedes 31 nach hinten folgt das axial verschiebbar im Handstück 3 gelagerte Widerlager 28 dem Schieber 33 selbsttätig unter der Federspannung, wodurch sich die Vorspannung der Feder 27 verringert. Dabei kann die Veränderung des Drehmomentwertes stufenlos oder in Stufen erfolgen. Zur Feststellung in der jeweiligen Verstellposition der vorhandenen Schiebeführung kann z. B. ein Klemm- oder Feststellelement vorgesehen sein. Bei der vorliegenden Ausgestaltung ist das Einstellglied 31 ein die Griffhülse umgebender Drehring 31a, der ein Innengewinde aufweist, mit dem er auf einem Außengewinde der Griffhülse aufgeschraubt ist. Das Verbindungsglied 32 ist durch einen Stift oder eine Schraube gebildet, die die Griffhülse in einer Ausnehmung 34 durchsetzt und in den vorzugsweise als Ring ausgebildeten und den Drehwellenabschnitt 10 mit Bewegungsspiel umgebenden Schieber 33 einfaßt. Die Ausnehmung 34 kann der Steilheit des Gewindes 35 folgen oder in Umfangsrichtung verlaufen. Die Steilheit des Gewindes 35 ist so groß zu bemessen, daß die Bewegungslänge in Umfangsrichtung ausreicht, um das Widerlager 28 und den Drehmomentwert in gewünschter Weise zu verändern.

Die Feder 27 kann zwischen zwei Wälz- bzw. Kugellagern 28a, 28b eingespannt sein, die beide in der Griffhülse und auf dem Antriebswellenabschnitt 10 mit geringem Bewegungsspiel axial verschiebbar gelagert sind, wobei das vordere Wälzlager 28b fest auf dem Antriebswellenabschnitt 10 und/oder an der Kupplungsscheibe 14a angeordnet sein kann.

Es ist vorteilhaft, dem Einstellglied 31 eine Skalierung mit einer Skala 36a und einer Gegenskalierung oder einem Index 36b auf der Mantelfläche der Griffhülse und am Einstellglied 31 zuzuordnen, die bestimmte Einstellungen der Drehmomentwert-Veränderung zum einen ermöglicht und zum anderen ablesen läßt. Bei einer Verstellbarkeit des Einstellgliedes 31 in axialer Richtung ist die Skalierung ebenfalls axial gerichtet. Beim Drehring 31a ist die Skalierung in Umfangsrichtung gerichtet. Aufgrund des Gewindeeingriffs zwischen dem Drehring 31a und der Griffhülse ist das so gebildete mechanische Getriebe selbsthemmend, so daß eine unbeabsichtigte Verstellung der Einstellung nicht erfolgt.

Zwecks Durchführung oder Anpassung an gleiche oder unterschiedliche Behandlungs- oder Bearbeitungsarten mit Werkzeugen gleicher oder unterschiedlicher Formen und/oder Querschnitte und/oder Größe und/oder Festigkeit gibt es Werkzeuge 6, die zur besseren Unterscheidung mit einer farblichen oder anderen Markierung, z.B. Markierungszeichen, gekennzeichnet sind, insbesondere an ihren vorzugsweise gleich großen Schäften. Es ist deshalb vorteilhaft und dient einer einfacheren Handhabung, wenn am Einstellglied 31 oder der Griffhülse eine oder mehrere farbliche oder andere Markierungen 36c auf dem Einstellweg vorgesehen sind, die so angeordnet sind, daß bei Positionsübereinstimmung mit einem auf dem jeweils anderen Teil angeordneten Index 36d ein Drehmomentwert eingestellt ist, der dem Werkzeug 6 gleicher Farbe oder gleicher Markierung entspricht. Hierdurch ist eine vereinfachte Einstellung des Handstücks 2 an die Größe oder an die Art von wenigstens einem Werkzeug 6 gegeben. Bei Überlastungen, z.B. dann, wenn das Werkzeug 6 festsitzt, rutscht oder öffnet die Überlastkupplung 14, wodurch das übertragbare Drehmoment begrenzt ist und das Werkzeug 6 oder ein damit beanspruchtes Bauteil, z.B. ein Implantat, vor Überlastung, insbesondere Bruch, geschützt ist. Die Markierung 36c und der Index 36d können in die Skalierung 36a, 36b integriert sein. Es ist im weiteren vorteilhaft, die wengistens eine Markierung 36c so anzuordnen, daß sie mit einem oder den zugehörigen Raststellen 51a, 51b, 51c übereinstimmt, wodurch die Einstellung weiter vereinfacht wird.

Vorzugsweise besteht der Schieber 33 aus einem Außenring 33a und einem Innenring 33b, die durch ein Gewinde 37 koaxial in Eingriff stehen, so daß durch axiales Schrauben des Innenrings 33b relativ zum Außenring 33a eine gewünschte Nullpunkteinstellung und somit ein bestimmtes Ausgangs-Drehmoment für die Überlastkupplung 14 einstellbar und somit die Überlastkupplung 14 justierbar ist. Aufgrund des Gewindeeingriffs ist auch diese Einstellung selbsthemmend, so daß eine unbeabsichtigte Verstellung verhindert ist. Zum Verstellen kann der Innenring 33b an seiner Hinterseite Angriffselemente, z. B. Ausnehmungen, für ein passendes Werkzeug (nicht dargestellt) aufweisen, mit dem er verdrehbar ist.

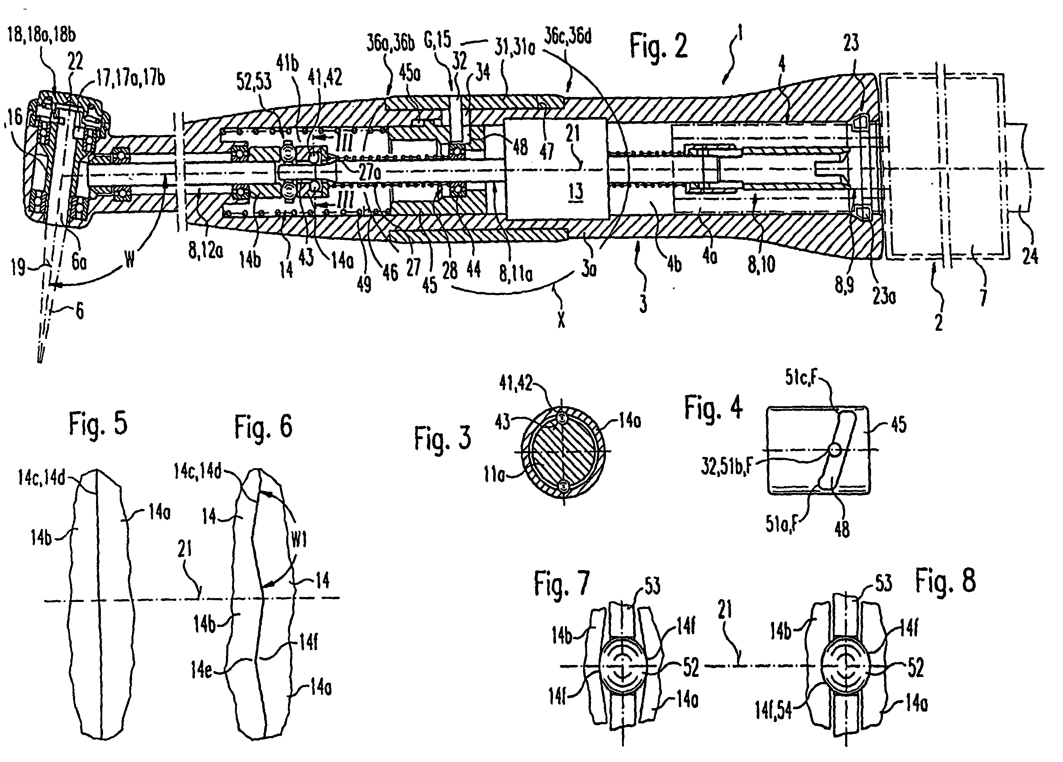

Das Ausführungsbeispiel nach Fig. 2 bis 7, bei dem gleiche oder vergleichbare Teile mit gleichen Bezugszeichen versehen sind, unterscheidet sich vom vorbeschriebenen Ausführungsbeispiel in mehrfacher Hinsicht. Das Untersetzungsgetriebe 13 kann im Handstück 3 bzw. in der Griffhülse angeordnet sein, hier in ihrem hinteren oder mittleren Bereich, insbesondere im Bereich des zweiten Antriebswellenabschnitts 10. Die Überlastkupplung 14 und die Einstellvorrichtung 15 sind bezüglich des Untersetzungsgetriebes 13 nach vorne versetzt angeordnet. Bei dieser Ausgestaltung ist ein sich gerade erstreckendes Handstück 3 vorgesehen, jedoch kann auch hier eine Winkelform vorgesehen sein. Der Winkel W kann auch hier sowohl bei gerader als auch Winkelform etwa 90° bis 100° oder etwa 90° oder etwa 100° betragen. Die Überlastkupplung 14 ist ebenfalls zwischen zwei Antriebswellenabschnitten angeordnet, die hier mit 11a und 12a bezeichnet sind.

Bei der vorliegenden Ausgestaltung sitzt die axial feste bzw. vordere Kupplungsscheibe 14b mit einer Bohrung auf dem hinteren Ende des Antriebswellenabschnitts 12a drehfest, wobei sie mit ihrer nach hinten gerichteten Stirnfläche die zugehörige Kupplungs- bzw. Reibfläche 14d bildet. Die hintere Kupplungsscheibe 14a mit ihrer Kupplungs- bzw. Reibfläche 14c ist mit einer Bohrung axial verschiebbar auf dem vorderen Endbereich des Antriebswellenabschnitts 11a gelagert, jedoch durch eine Drehmitnahmeverbindung drehfest mit dem Antriebswellenabschnitt 11a verbunden. Hierzu können wenigstens ein Mitnehmeransatz im zentralen Loch der Kupplungsscheibe 14a oder wenigstens eine Mitnehmerkugel 41 dienen, der bzw. die in eine Längsnut 43 im Antriebswellenabschnitt 11 mit Bewegungsspiel einfaßt, die am vorderen Ende des Antriebswellenabschnitts 11 axial ausmündet. Die Kugel 41 sitzt in einer radial nach innen offenen Ausnehmung 42 in der Kupplungsscheibe 14a. Es können einander gegenüberliegend zwei oder mehrere Mitnehmeransätze oder Kugeln 41 und Längsnuten 43 im Sinne einer Mehrkeil-Wellenverbindung vorgesehen sein. Zwischen der hier auf dem Antriebswellenabschnitt 11a angeordneten Feder 27 und der Kupplungsscheibe 14a kann eine Scheibe 27a angeordnet sein.

Das Widerlager 28 wird hier durch einen Lagerring, insbesondere ein Wälz- bzw. Kugellager 44 gebildet, das in einem zylindrischen Schieber 45 gelagert ist, der mit geringem Bewegungsspiel in einer Bohrung 46 in der Griffhülse 3a axial verschiebbar und nicht drehbar gelagert ist. Hierzu kann eine Keilnutverbindung z.B. mit einem Ansatz 45a am Umfang des Schiebers 45 vorgesehen sein, der mit Bewegungsspiel in eine Längsnut in der Innenwand der Griffhülse 3a einfaßt. Auch bei dieser Ausgestaltung ist das Einstellglied 31 durch einen Drehring 31a gebildet, der jedoch hohlzylindrisch ausgebildet ist und mit seinem Innenumfang auf einem zylindrischen Lagerabschnitt 47 der Griffhülse 3a drehbar gelagert ist. Im Gegensatz zum vorbeschriebenen Ausführungsbeispiel ist hier das eine Drehbewegung in eine Axialbewegung umwandelnde Getriebe G durch eine schräg oder kurvenförmig verlaufende Einstellnut 48 im Schieber 45 gebildet, wobei das z. B. ebenfalls durch einen Stift oder eine Schraube gebildete und am Drehring 31a befestigte Verbindungsglied 32 ein entsprechend groß bemessenes und die erforderliche Bewegung ermöglichendes Loch 3b in der Griffhülse 3a durchfaßt und in die Einstellnut 48 mit Bewegungsspiel einfaßt. Hier bilden der Schieber 45 und das Wälzlager 44 eine Bewegungseinheit, wobei letzteres auf dem Antriebswellenabschnitt 11a mit geringem Bewegungsspiel verschiebbar gelagert ist und der Außenring des Wälzlagers an einer hinteren Schulterfläche im Schieber 45 anliegen kann. Die Feder 27 stützt sich rückseitig mittelbar über das Wälzlager 44 am Schieber 45 ab. Des weiteren kann eine vorzugsweise durch eine Druck-Wendelfeder gebildete Feder 49 vorgesehen sein, die am vorderen Ende der Bohrung 46 in der Griffhülse abgestützt ist und den Schieber 45 nach hinten vorspannt. Bei einem manuellen Verdrehen des Drehrings 31a wird der Schieber 45 bzw. das Widerlager 28 aufgrund des schrägen oder kurvenförmigen Verlaufs der Einstellnut 48, die eine Führung für das Verbindungsglied 32 bildet, axial verstellt und somit die Vorspannung der Feder 27 und der Drehmomentwert entsprechend verändert und eingestellt, nämlich vergrößert oder verkleinert.

Bei einem Verlauf der Einstellnut 48 ohne Selbsthemmung und/oder dann, wenn auf dem Einstellweg bestimmte Einstellstufen durch Druckpunkte spürbar sein sollen, sind in der Einstellnut 48 Rastausnehmungen 51a, 51b, 51c zugeordnet, in die das stiftförmige Verbindungsglied 32 unter der Vorspannung der Feder 49 einrastet, wodurch die Einstellung manuell überdrückbar festgestellt ist oder wodurch die Einstellstufen manuell spürbar sind. Hierdurch ist eine lösbare bzw. überdrückbare Feststellvorrichtung F für die Einstellvorrichtung 15 gebildet. Fig. 4 zeigt die Einstellnut 48 in der Abwicklung. Wie bereits die Markierung 36c, 36d können auch die Raststellen 51a, 51b, 51c in Positionen auf dem Verstellweg angeordnet sein, die bestimmten Drehmomentwerten, insbesondere der zugehörigen, unterschiedlichen Werkzeige 6, entsprechen. Vorzugsweise sind die Markierungen 36c den Raststellen 51a, 51b, 51c zugeordnet.

Auch bei der Ausgestaltung nach Fig. 2 kann eine Skalierung mit einer Skala 36a und einer einem Index 36b am Umfang der Griffhülse und des Drehrings 31a vorgesehen sein. Die Rastausnehmungen 51a, 51b, 51c können auch am linken Rand der Einstellnut 48 vorgesehen sein, wenn die Feder 49 nach vorne wirksam ist. Eine schräge oder kurvenförmige Einstellnut 48 kann auch am Drehring 31 für das Verbindungsglied 32 vorgesehen sein, wenn letzteres am Schieber 45 befestigt ist.

Gemäß Fig. 5 oder 6 liegen die Reibflächen 14c, 14d der Kupplungsscheiben 14a, 14b aneinander an, wobei die Reibflächen 14c, 14d gemäß Fig. 5 in einer rechtwinklig zur Längsmittelachse 21 gerichteten Querebene angeordnet sind. Gemäß Fig. 6 sind die Reibflächen 14c, 14d mit ineinander passenden axialen Erhebungen 14e und Vertiefungen 14f ausgebildet, deren Flankenflächen radial verlaufen und gleiche stumpfe Winkel W1 von etwa 135° bis 170° einschließen. Die Form der Erhebungen 14e und Vertiefungen 14f oder ihre Flanken braucht nicht keilförmig zu sein. Es kann auch eine trapezförmige Form oder eine gerundete bzw. kurvenförmige Form vorgesehen sein.

Bei der Ausgestaltung nach Fig. 7 sind zwischen den Reibflächen 14c, 14d Wälzelemente 52, insbesondere Kugeln, angeordnet, die in einem Käfig 53 gehalten sind. Beim Ausführungsbeispiel gemäß Fig. 8 sind in beiden Reibflächen 14c, 14d einander gegenüberliegend Kalotten 54 angeordnet, deren Form und Größe vorzugsweise der Form und Größe der Wälzelemente 52, hier der Kugeln, entspricht.

Während bei der Ausgestaltung die axiale Verschiebbarkeit der Kupplungsscheibe 14a gering sein kann, muß bei den Ausgestaltungen nach Fig. 6 bis 8 das Maß der axialen Verschiebung gleich oder größer sein als die axiale Tiefe der Vertiefungen 14f (Fig. 6) oder größer als die Summe eines Paares Vertiefungen 14f gemäß Fig. 7 und 8. Bei der Ausgestaltung nach Fig. 5 erfolgt eine kraftschlüssige Drehmoment-Übertragung. Dagegen erfolgt bei den Ausgestaltungen nach Fig. 6 bis 8 eine forcierte formschlüssige Drehmoment-Übertragung bzw. eine Kombination aus kraft- und formschlüssiger Drehmoment-Übertragung.

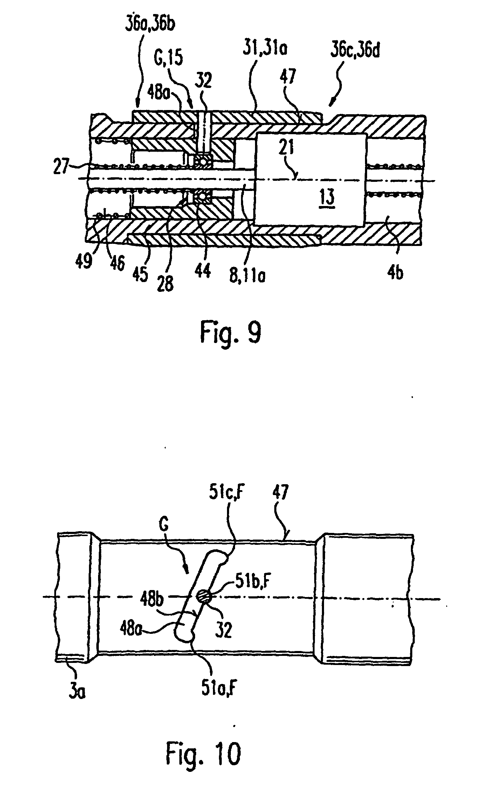

Bei dem Ausführungsbeispiel nach Fig. 9 und 10, bei dem gleiche oder vergleichbare Teile mit gleichen Bezugszeichen versehen sind, ist die das Getriebe G bildende Einstellnut 48a in der Griffhülse 3a angeordnet. Im Gegensatz zum Ausführungsbeispiel nach Fig. 4 mit einer vorderseitigen schrägen oder kurvenförmigen Einstellfläche 48b weist die Einstellnut 48a eine rückseitige schräge oder kurvenförmige Einstellnut 48b auf, an der bei einer Verdrehung des Einstellgliedes 31 das Verbindungsglied 32 entlanggleitet, wobei das Einstellglied 31 und der Schieber 45 je nach Drehrichtung des Einstellgliedes 31 nach vorne oder nach hinten verstellbar sind bzw. verstellt werden. Die Einstellnut 48 bzw. 48a kann in ihrer axialen Abmessung mit Bewegungsspiel an die axiale Querschnittsabmessung des Verbindungsglieds 32 angepaßt sein und somit beidseitig Führungsflächen aufweisen im Sinne einer beidseitig wirksamen Führungskulisse, oder sie kann auch durch ein in axialer Richtung größer als das Verbindungsglied 32 bemessenes Loch gebildet sein, wobei die Feder 27 die Rückstellungsbewegung nach hinten erzeugt. Auch bei der Ausgestaltung nach Fig 9 und 10 können in der Einstellfläche 48b die Rastausnehmungen 51a bis z.B. 51c vorgesehen sein, um überdrückbare Raststellen zu bilden. Zur Verbindung des Verbindungsgliedes 32 mit dem Schieber 45 kann eine feste Verbindung vorgesehen sein, z.B. kann der Stift in ein Loch im Schieber 45 fest eingepreßt oder eingeklebt sein oder das Verbindungsglied 32 kann auch an der Rückseite des Schieber 45 anliegen. Einer Drehsicherung für den Schieber 45 ( s. Bezugszeichen 45a) bedarf es nicht. Die Einstellnut 48a kann sich gerade oder kurvenförmig erstrecken.

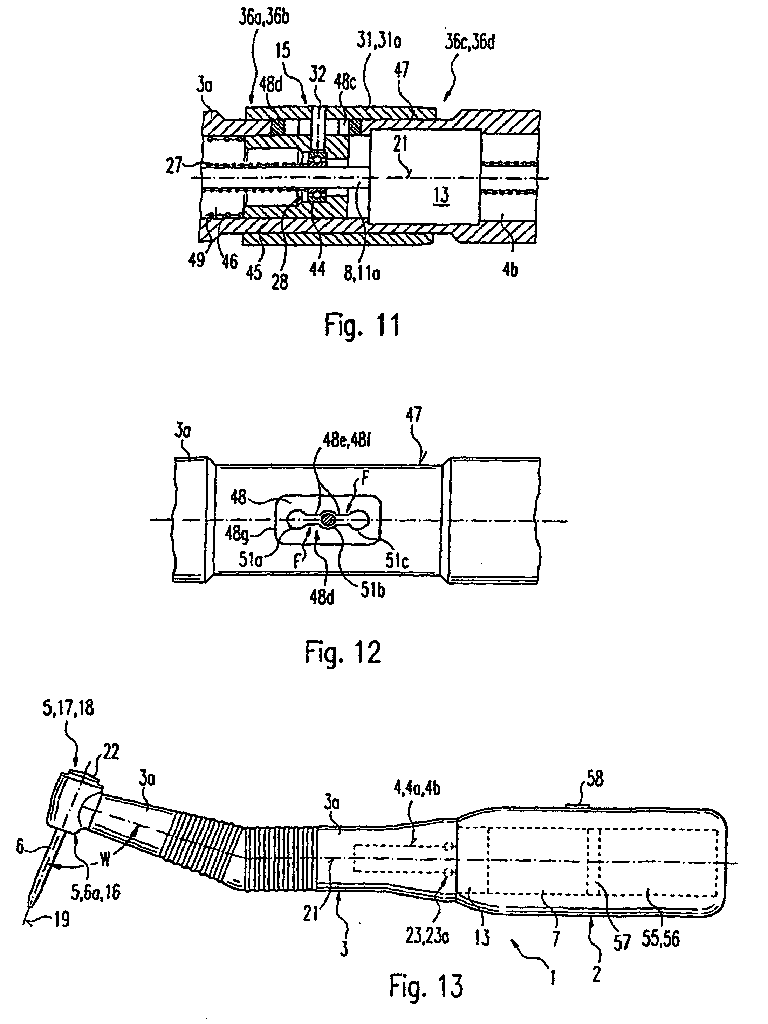

Beim Ausführungsbeispiel nach Fig. 11 und 12, dem gleiche oder vergleichbare Teile ebenfalls mit gleichen Bezugszeichen versehen sind, unterscheidet sich vom Ausführungsbeispiel nach Fig. 9 und 10 dadurch, daß die Einstellnut 48c in der Griffhülse 3a axial angeordnet ist und eine Feststellvorrichtung F zum Feststellen des Einstellgliedes 31 stufenlos oder in Stufen vorgesehen ist. Eine in Stufen wirksame Feststellvorrichtung F kann durch Klemm- oder Verrastungsstellen in der vorzugsweise eine Führungskulisse mit beiderseitigen Führungsflächen 48e bildenden Einstellnut 48c gebildet sein. Die Raststellen können durch seitliche Ausnehmungen gebildet sein. Bei der vorliegenden Ausgestaltung weist die Einstellnut 48c ein- oder beidseitig Rastausnehmungen 51a bis z.B. 51 c auf, wobei dazwischen verjüngte Stellen 48f angeordnet sind durch die das Verbindungsglied 32 mit einem gewissen axialen manuellen Kraftaufwand durchdrückbar und somit die Verrastung überdrückbar ist. Hierbei kann das Verbindungsglied 32 und/oder der Rand der Einstellnut 48c aus elastisch verformbaren Material, vorzugsweise Gummi oder Kunststoff, bestehen. Bei dieser Ausgestaltung kommt das Getriebe G in Fortfall. Es ist auch möglich, die Einstellnut 48a oder 48c in einem z. B. aus Gummi oder Kunststoff bestehenden Einsatzteil 48d auszubilden, das in einer Ausnehmung 48g entsprechender Form und Größe in der Griffhülse 3a sitzt.

Bei der Ausgestaltung nach Fig. 9 und 10 führt das Einstellglied Bewegungen in Umfangsrichtung und in axialer Richtung aus, während es bei der Ausgestaltung nach Fig. 11 und 12 Bewegungen nur in axialer Richtung ausführt oder dann, wenn die Rastausnehmungen 51a bis 51c eine größere seitliche Tiefe aufweisen, auch Bewegungen in Umfangsrichtung ausführt. Entsprechend länger als das Einstellglied 31 ist der Lagerabschnitt 47 auszubilden.

Beim Ausführungsbeispiel nach Fig. 13, bei dem gleiche oder vergleichbare Teile mit gleichen Bezugszeichen versehen sind, ist das Behandlungsinstrument 1 als autarkes Instrument mit einer Aufnahmevorrichtung 55 für eine Batterie 56 zur Stromversorgung des Antriebsmotors 7 ausgebildet. Die Aufnahmevorrichtung 55 und die Batterie 56 sowie eine zugehörige elektronische Steuervorrichtung 57 können im Anschlußteil 2 z.B. hinter dem Antriebsmotor 7 angeordnet sein. Das Handstück 3 und die Schnellkupplung 4 können entsprechend dem vorgeschriebenen Ausführungsbeispiel ausgebildet sein.Hierdurch ist es möglich, das Handstück 3 einer Desinfektion oder Sterilisation gegebenenfalls bei erforderlicher Hitze durchzuführen und das Anschlußteil 2 auf andere Weise zu desinfizieren oder zu pflegen. Es ist auch möglich, das Handstück 3 ohne Schnellkupplung betriebsmäßig fest mit dem Anschlußteil 2 zu verbinden. Somit kann dieses Behandlungsinstrument 1 auch ein Handstück 3 bilden, in dessen hinteren Endbereich die Aufnahmevorrichtung 55, die Batterie 56 und die elektronische Steuervorrichtung 57 angeordnet sind. Die Anordnung ist vorzugsweise für eine aufladbare Batterie 56 mit einer von außen zugänglichen Kontaktanordnung zum Aufladen ausgebildet.

Bei allen vorbeschriebenen Ausführungsbeispielen ist es möglich, anstelle einer mechanischen Antriebs- oder Drehmomentbegrenzung eine elektrische Antriebs- oder Drehmomentbegrenzung vorzusehen, die z.B. nach Maßgabe der Stromstärke den Antriebsmotor 7 so steuert, daß ein bestimmtes maximales Moment nicht überschritten wird. Außerdem kann eine elektrische Einstellvorrichtung zur Veränderung dieses Momentwertes vorgesehen sein. Hierzu kann z.B. am Umfang des Anschlußteils 2 ein verstellbares Einstellglied 58 vorgesehen sein, an dem das veränderliche Moment manuell verstellbar ist.

Eine elektrische Steuervorrichtung zur Begrenzung des übertragbaren Antriebsmomentwertes oder auch zur Einstellung dieses Wertes kann bei allen vorbeschriebenen Ausführungsbeispielen gemäß Fig. 1 und 2 z.B. in das nicht dargestellte Steuergerät integriert sein.

Das erfindungsgemäße Behandlungsinstrument 1 oder Handstück 3 eignet sich vorzüglich nicht nur zur Aufbereitung eines Zahn-Wurzelkanals sondern auch zum Ein- und/oder Ausschrauben eines Implantats in und/oder aus einem Knochen oder einer Prothese, insbesondere Kierferknochen, oder zum Ein- und/oder Ausschrauben eines Verankerungselements in und/oder aus einem Implantat. In diesem Falle ist ein Werkzeug 6 oder Werkzeugset mit individuell unterschiedlichen Werkzeugen vorgesehen, wobei das oder die Werkzeuge 6 jeweils am vorderen Ende ein mit dem Implantat oder dem Verankerungselement zusammenwirkendes unrundes Drehmitnahmeelement aufweist, das formschlüssig mit einem Gegenelement am Implantat oder am Verankerungselement zusammenwirkt. Damit ein- und ausgeschraubt werden kann, ist dem Antrieb ein z. B. am Instrument 1 oder am Handstück 3 oder an einem Fußschalter angeordnetes elektrisches oder mechanisches Schalterelement vorgesehen.

## Patentansprüche

1. Handstück (3) für medizinische Zwecke, insbesondere für eine ärztliche oder zahnärztliche Behandlungseinrichtung, vorzugsweise für eine spanabhebende Bearbeitung eines Zahn-Wurzelkanals oder zur Ausübung eines Antriebs oder Drehantriebs mit
- einem im vorderen Endbeteich des Handstücks (3) angeordneten Werkzeughalter (5),
- einer Haltekupplung (16) zum lösbaren Fixieren des Werkzeugs (6) im Werkzeughalter (5)
- und einer sich längs durch das Handstück (3) erstreckenden Antriebsverbindung (8) für einen Dreh- und/oder Hubantrieb des Werkzeughalters (5),
- wobei in der Antriebsverbindung (8) eine Überlastkupplung (14) angeordnet ist, die das übertragbare Antriebs- oder Drehmoment auf einen maximalen Antriebs- oder Drehmomentwert begrenzt,
und wobei ein Kupplungsteil (14a) axial verschiebbar gelagert und durch eine Federkraft (27) in seine Kupplungsstellung vorgespannt ist,
**dadurch gekennzeichnet,**
**daß** der Drehmomentwert durch eine Einstellvorrichtung (15) mit einem von außen zugänglichen Einstellglied (31) manuell stufenlos oder in Stufen veränderlich ist und daß mit der Einstellvorrichtung (15) die Spannung der Federkraft veränderlich ist.

2. Handstück (3) für medizinische Zwecke, insbesondere für eine ärztliche oder zahnärztliche Behandlungseinrichtung, vorzugsweise für eine spanabhebende Bearbeitung eines Zahn-Wurzelkanals oder zur Ausübung eines Antriebs oder Drehantriebs mit
- einem im vorderen Endbereich des Handstücks (3) angeordneten Werkzeughalter (5),
- einer Haltekupplung (16) zum lösbaren Fixieren des Werkzeugs (6) im Werkzeughalter (5)
- und einer sich im Handstück (3) nach vorne erstreckenden Antriebsverbindung (8) für einen Dreh- und/oder Hubantrieb des Werkzeughalters (5),
**dadurch gekennzeichnet,**
**daß** im hinteren Endbereich (2) des Handstücks (3) eine Aufnahmevorrichtung (55) für eine Batterie (56), eine elektronische Steuervorrichtung (57) und ein elektrischer Antriebsmotor (7) angeordnet sind,
**daß** die elektronische Steuereinrichtung (57) das übertragbare Antriebs- oder Drehmoment auf einen maximalen Antriebs- oder Drehmomentwert begrenzt,
und **daß** der Drehmomentwert durch eine Einstellvorrichtung (15) mit einem von außen zugänglichen Einstellglied (31) manuell stufenlos oder in Stufen veränderlich ist.

3. Handstück (3) für medizinische Zwecke, insbesondere für eine ärztliche oder zahnärztliche Behandlungseinrichtung, vorzugsweise für eine spanabhebende Bearbeitung eines Zahn-Wurzelkanals oder zur Ausübung eines Antriebs oder Drehantriebs mit
- einem im vorderen Endbereich des Handstücks (3) angeordneten Werkzeughalter (5),
- einer Haltekupplung (16) zum lösbaren Fixieren des Werkzeugs (6) im Werkzeughalter (5)
- einer sich im Handstück (3) nach vorne erstreckenden Antriebsverbindung (8) für einen Dreh- und/oder Hubantrieb des Werkzeughalters (5),
- und einer in der Antriebsverbindung (8) angeordneten Überlastkupplung (14), die das übertragbare Antriebs- oder Drehmoment auf einen maximalen Antriebs- oder Drehmomentwert begrenzt,
**dadurch gekennzeichnet,**
- **daß** im hinteren Endbereich (2) des Handstücks (3) eine Aufnahmevorrichtung (55) für eine Batterie (56), eine elektronische Steuervorrichtung (57) und ein elektrischer Antriebsmotor (7) angeordnet sind,
und **daß** der Drehmomentwert durch eine Einstellvorrichtung (15) mit einem von außen zugänglichen Einstellglied (31) manuell stufenlos oder in Stufen veränderlich ist.

4. Händstück nach Anspruch 1 oder 3,
**dadurch gekennzeichnet,**
**daß** das Handstück (3) durch eine Schnellkupplung (4) lösbar mit einem hinteren Anschlußteil (2, Fig. 1 und 2) verbunden ist.

5. Händstück nach Anspruch 2,
**dadurch gekennzeichnet,**
**daß** das Handstück (3) durch eine Schnellkupplung (4) lösbar mit einem die Aufnahmevorrichtung (55), eine elektronische Steuereinrichtung (57) und einen elektronischen Antriebsmotor (7) aufweisenden hinteren Endbereich (2, Fig. 13) verbunden ist.

6. Händstück nach Anspruch 1,
**dadurch gekennzeichnet,**
**daß** die Überlastkupplung (14) im sich zum Werkzeughalter (5) erstreckenden Antriebsverbindungsabschnitt (10, 11, 11a, 12a) der Antriebsverbindung (8) angeordnet ist.

7. Händstück nach einem der vorherigen Ansprüche 1, 3 und 4,
**dadurch gekennzeichnet,**
**daß** die Überlastkupplung (14) im mittleren oder hinteren Bereich des länglichen Handstücks (3) angeordnet ist.

8. Handstück nach einem der vorherigen Ansprüche,
**dadurch gekennzeichnet.**
**daß** ein vorzugsweise untersetzendes Getriebe (13) vorgesehen ist, das im Handstück (3), insbesondere in dessen mittleren oder hinteren Bereich, oder im Anschlußteil (2) angeordnet ist.

9. Handstück nach Anspruch 8,
**dadurch gekennzeichnet,**
**daß** die Überlastkupplung (14) dem Getriebe (13) auf dessen dem Werkzeughalter (5) zugewandten Seite vorgeordnet ist.

10. Händstück nach einem der vorherigen Ansprüche 1, 3 und 4 sowie 6 bis 9,
**dadurch gekennzeichnet,**
**daß** die Überlastkupplung (14) zwei drehbar gelagerte Kupplungsteile (14a, 14b) aufweist, die koaxial zueinander angeordnet sind und jeweils eine rotationssymmetrische Reibfläche (14c, 14d) aufweisen, an denen die Kupplungsteile (14a, 14b) mit einer vorzugsweise federelastischen Vorspannung aneinanderliegen.

11. Händstück nach Anspruch 10,
**dadurch gekennzeichnet,**
**daß** die Reibflächen (14c, 14d) sich in einer zur Längsmittelachse (21) der Kupplungsteile (14a, 14b) rechtwinkligen Ebene erstrecken.

12. Handstück nach einem der vorherigen Ansprüche 3 und 4 sowie 6 bis 11,
**dadurch gekennzeichnet,**
**daß** ein Kupplungsteil (14a) axial verschiebbar gelagert und durch eine Federkraft (27) in seine Kupplungsstellung vorgespannt ist.

13. Handstück nach einem der vorherigen Ansprüche 1, 3 und 4 sowie 6 bis 12,
**dadurch gekennzeichnet,**
**daß** die Überlastkupplung (14) zwei drehbar gelagerte Kupplungsteile (14a, 14b) aufweist, die koaxial zueinander angeordnet sind und an ihren einander zugewandten Seiten in Umfangsrichtung einander abwechselnd folgende und zueinander passende Vertiefungen (14f) und Erhebungen (14e) mit axial schrägen oder gerundeten Flanken aufweisen, wobei ein oder beide Kupplungsteile (14a) zum Zweck der Entkupplung elastisch nachgiebig gelagert ist bzw. sind.

14. Handstück nach einem der vorherigen Ansprüche 1, 3 und 4 sowie 6 bis 13,
**dadurch gekennzeichnet,**
**daß** die Überlastkupplung (14) zwei drehbar gelagerte Kupplungsteile (14a, 14b) aufweist, die koaxial zueinander angeordnet sind und an ihren einander zugewandten Seiten in Umfangsrichtung einander folgende und einander gegenüberliegende Vertiefungen (14f) mit axial schrägen oder gerundeten Flanken aufweisen, wobei ein oder beide Kupplungsteile (14a) zum Zweck der Entkupplung elastisch nachgiebig gelagert ist bzw. sind und wobei zwischen den Kupplungsteilen (14a, 14b) Wälzkörper (52), insbesondere Kugeln gelagert sind, deren Queischnittsgröße größer ist als die Summe der Tiefe der Vertiefungen (14f).

15. Handstück nach Anspruch 13 oder 14,
**dadurch gekennzeichnet,**
**daß** der von den Flanken eingeschlossene Winkel (W1) stumpf ist und vorzugsweise etwa 135 bis 170° beträgt.

16. Handstück nach Anspruch 14 oder 15,
**dadurch gekennzeichnet,**
**daß** die Vertiefungen (14f) durch vorzugsweise an die Form und Größe der Wälzkörper (52) angepaßte insbesondere kugelabschnittförmige Kalotten (54) gebildet sind.

17. Handstück nach einem der vorherigen Ansprüche,
**dadurch gekennzeichnet,**
**daß** das Einstellglied (31) in Umfangsrichtung und/oder axial verstellbar ist und vorzugsweise am Umfang des Handstücks (3) angeordnet ist.

18. Handstück nach einem der vorherigen Ansprüche,
**dadurch gekennzeichnet,**
**daß** der Einstellvorrichtung (15) oder dem Einstellglied (31) eine Feststellvorrichtung (F) zum lösbaren Arretieren in der jeweils eingestellten Position zugeordnet ist.

19. Handstück nach Anspruch 18,
**dadurch gekennzeichnet,**
**daß** die Feststellvorrichtung (F) durch eine Rastvorrichtung gebildet ist, die vorzugsweise zu ihrer Lösung manuell überdrückbar ist.

20. Handstück nach einem der Ansprüche 12 bis 19,
**dadurch gekennzeichnet,**
**daß** mit der Einstellvorrichtung (15) die Verspannung der Federkraft veränderlich ist.

21. Handstück nach einem der vorherigen Ansprüche,
**dadurch gekennzeichnet,**
**daß** das vorzugsweise durch einen Schieber oder Drehring gebildete Einstellglied (31) durch ein radiales Verbindungsglied (32) mit der Einstellvorrichtung (15) verbunden ist.

22. Händstück nach einem der vorhergehenden Ansprüche,
**dadurch gekennzeichnet,**
**daß** die Einstellvorrichtung (15) einen mit dem Drehring (31a) in Antriebsverbindung stehenden Gewindetrieb (35) oder Kurventrieb (48) aufweist.

23. Handstück nach einem der vorherigen Ansprüche,
**dadurch gekennzeichnet,**
**daß** auf dem Verstellweg des Einstellglieds (31) eine Skalierung (36a, 36b) und/oder Markierung (36c) jeweils mit einer Gegenskalierung oder mit einer Gegenmarkierung bzw. einem Index und/oder eine oder mehrere Raststellen (51a, 51b, 51c) am Einstellglied (31) und einem ihm benachbarten Teil, insbesondere dem das Einstellglied (31) tragenden Teil, angeordnet ist bzw. sind.

24. Handstück nach Anspruch 23,
**dadurch gekennzeichnet,**
**daß** die Markierung (36c) unterschiedliche Zeichen oder Farben aufweist.

25. Handstück nach einem der vorherigen Ansprüche,
**dadurch gekennzeichnet,**
**daß** mehrere unterschiedliche Werkzeuge (6) vorgesehen sind, die sich z.B. bezüglich ihrer Form und/oder Größe und/oder Festigkeit voneinander unterscheiden.

26. Handstück nach Anspruch 25,
**dadurch gekennzeichnet,**
**daß** die Werkzeuge (6), vorzugsweise jeweils am Werkzeugschaft (6a), eine Markierung (36c) aufweisen, wobei die Position der Markierung (36c) und/oder der Raststellen (51a, 51b, 51c) auf dem Verstellweg einem bestimmten, z.B. größtmöglichen, Antriebs- oder Drehmomentwert des zugehörigen Werkzeugs (6) entspricht.

## Claims

1. Handpiece (3) for medical purposes, in particular for a medical or dental treatment facility, preferably for a material removing working of a tooth root canal or for exercising a drive or rotary drive having
- a tool holder (5) arranged in the forward end region of the handpiece (3),
- a retention coupling (16) for the releasable fixing of the tool (6) in the tool holder (5),
- and a drive connection (8), extending longitudinally through the handpiece (3), for a rotary and/or stroke drive of the tool holder (5),
- there being arranged in the drive connection (8) an overload coupling (14) which restricts the transmissible driving moment or torque to a maximum driving moment or torque value,
and whereby a coupling part (14a) is axially displaceably mounted and biassed into its coupling position by means of a spring force (27),
**characterised in that**,
the torque value is manually variable, steplessly or in steps, by means of a setting device (15) having an externally accessible setting member (31) and **in that** the tension of the spring force is variable with the setting device (15).

2. Handpiece (3) for medical purposes, in particular for a medical or dental treatment facility, preferably for a material removing working of a tooth root canal or for exercising a drive or rotary drive having
- a tool holder (5) arranged in the forward end region of the handpiece (3),
- a retention coupling (16) for the releasable fixing of the tool (6) in the tool holder (5),
- and a drive connection (8), extending forwardly in the handpiece (3), for a rotary and/or stroke drive of the tool holder (5),
**characterised in that**,
in the rearward end region (2) of the handpiece (3) there are arranged a receiving device (55) for a battery (56), an electronic control device (57) and an electric drive motor (7),
**in that** the electrical control device (57) limits the transmissible drive moment or torque to a maximum drive moment or torque value,
and **in that** the torque value is manually variable, steplessly or in steps, by means of a setting device (15) having an externally accessible setting member (31).

3. Handpiece (3) for medical purposes, in particular for a medical or dental treatment facility, preferably for a material removing working of a tooth root canal or for exercising a drive or rotary drive having
- a tool holder (5) arranged in the forward end region of the handpiece (3),
- a retention coupling (16) for the releasable fixing of the tool (6) in the tool holder (5),
- a drive connection (8), extending forwardly in the handpiece (3), for a rotary and/or stroke drive of the tool holder (5),
- and an overload coupling (14), arranged in the drive connection (8), which restricts the transmissible driving moment or torque to a maximum driving moment or torque value,
**characterised in that**,
in the rearward end region (2) of the handpiece (3) there are arranged a receiving device (55) for a battery (56), an electronic control device (57) and an electric drive motor (7),
and **in that** the torque value is manually variable, steplessly or in steps, by means of a setting device (15) having an externally accessible setting member (31).

4. Handpiece according to claim 1 or 3,
**characterised in that**,
the handpiece (3) is releasably connected with a rearward connection part (2, Fig. 1 and 2) by means of a quick coupling (4).

5. Handpiece according to claim 2,
**characterised in that**,
the handpiece (3) is releasably connected by means of a quick coupling (4) with a rearward end region (2, Fig. 13) having the receiving device (55), an electronic control device (57) and an electronic drive motor (7).

6. Handpiece according to claim 1,
**characterised in that**,
the overload coupling (14) is arranged **in that** drive connection section (10, 11, 11a, 12a) of the drive connection (8) which extends to the tool holder (5).

7. Handpiece according to any of preceding claims 1, 3 and 4,
**characterised in that**,
the overload coupling (14) is arranged in the middle or rearward region of the elongate handpiece (3).

8. Handpiece according to any preceding claim,
**characterised in that**,
there is provided a transmission (13), preferably a step-down transmission, that is arranged in the handpiece (3) in particular in its middle or rearward region, or in the connection part (2).

9. Handpiece according to claim 8,
**characterised in that**,
the overload coupling (8) is arranged before the transmission (13) on the side thereof towards the tool holder (5).

10. Handpiece according to any of preceding claims 1, 3 and 4, and 6 to 9,
**characterised in that**,
the overload coupling (14) has two rotatably mounted coupling parts (14a, 14b) which are arranged coaxially of one another and have each a rotationally symmetric friction surface (14c, 14d) at which the coupling parts (14a, 14b) bear on one another with a preferably spring elastic biassing.

11. Handpiece according to claim 10,
**characterised in that**,
the friction surfaces (14c, 14d) extend in a plane at right angles to the longitudinal middle axis (1) of the coupling parts (14, 14b).

12. Handpiece according to any of preceding claims 3 and 4, and 6 to 11,
**characterised in that**,
one coupling part (14a) is axially displaceably mounted and biassed into its coupling position by means of a spring force (27).

13. Handpiece according to any of preceding claims 1, 3 and 4, and 6 to 12,
**characterised in that**,
the overload coupling (14) has two rotatably mounted coupling parts (14a, 14b) which are arranged coaxially of one another and have at their mutually facing sides depressions (14f) and elevations (14e) following one another alternately in the circumferential direction and matching one another, having axially oblique or rounded flanks, whereby one or both coupling parts (14a) is or are, for the purpose of decoupling, elastically yieldingly mounted.

14. Handpiece according to any preceding claims 1, 3 and 4, and 6 to 13,
**characterised in that**,
the overload coupling (14) has two rotatably mounted coupling parts (14a, 14b) which are arranged coaxially of one another and have on their mutually facing sides depressions (14f) following one another in the circumferential direction and opposite one another, having axially oblique or rounded flanks, whereby one or both coupling parts (14a) is or are, for the purpose of decoupling, elastically yieldingly mounted, and whereby between the coupling parts (14a, 14b) there are mounted roller bodies (52), in particular balls, the cross-sectional size of which is greater than the sum of the depths of the depressions (14f).

15. Handpiece according to claim 13 or 14,
**characterised in that**,
the angle (W1) included by the flanks is obtuse, and preferably about 135 to 170 degrees.

16. Handpiece according to claim 14 or 15,
**characterised in that**,
the depressions (14f) are formed by calottes (54), in particular ball section shaped, preferably adapted to the form and size of the roller bodies (52).

17. Handpiece according to any preceding claim,
**characterised in that**,
the setting member (31) is adjustable in the circumferential direction and/or axially and is preferably arranged on the circumference of the handpiece (3).

18. Handpiece according to any preceding claim,
**characterised in that**,
there is associated with the setting device (15) or the setting member (31) a fixing device (F) for the releasable locking of the respectively set position.

19. Handpiece according to claim 18,
**characterised in that**,
the fixing device (F) is formed by means of a latch device which preferably can be manually overcome for its release.

20. Handpiece according to any of claims 12 to 19,
**characterised in that**,
the tensioning of the spring force is variable with the setting device (15).

21. Handpiece according to any preceding claim,
**characterised in that**,
the setting member (31), preferably constituted by means of a slider or rotary ring, is connected with the setting device (15) by means of a radial connecting member (32).

22. Handpiece according to any preceding claim
**characterised in that**,
the setting device (15) has a thread drive (35) or a cam drive (48) standing in drive connection with the rotary ring (31a).

23. Handpiece according to any preceding claim,
**characterised in that**,
there is or are arranged on the path of adjustment of the setting member (31) a scaling (36a, 36b) and/or marking (36c) in each case with a counter-scaling or with a counter-marking or an index, and/or one or more latch points (51a, 51b, 51c) on the setting member (31) and a part neighbouring it, in particular the part carrying the setting member (31).

24. Handpiece according to claim 23,
**characterised in that**,
the marking (36c) has differing characters or colours.

25. Handpiece according to any preceding claim,
**characterised in that**,
a plurality of different tools (6) are provided, which differ from one another for example with regard to their shape and/or size and/or strength.

26. Handpiece according to claim 25,
**characterised in that**,
the tools (6) have, preferably in each case on the tool shaft (6a), a marking (36c), whereby the positioning of the marking (36c) and/or of the latch points (51a, 51b, 51c) on the path of adjustment correspond to a particular, for example large as possible, drive moment or torque value of the associated tool (6).

## Revendications

1. Pièce à main (3) pour applications médicales, en particulier pour un dispositif de traitement médical ou dentaire, de préférence pour la préparation d'un canal de racine dentaire ou pour l'exercice d'une commande de mouvement ou d'entraînement en rotation avec
- un porte-outil (5) disposé dans la zone d'extrémité avant de la pièce à main (3),
- un accouplement de retenue (16) pour la fixation amovible de l'outil (6) dans le porte-outil (5),
- et un raccordement d'entraînement (8) s'étendant longitudinalement à travers la pièce à main (3) pour un entraînement de rotation et/ou de levée du porte-outil (5),
- un accouplement de surcharge (14) étant disposé dans le raccord d'entraînement (8), qui limite le couple de rotation ou d'entraînement de mouvement transmissible à une valeur de couple de rotation ou d'entraînement de mouvement maximum,
et une partie d'accouplement (14a) étant montée de façon déplaçable axialement et étant précontrainte dans sa position d'accouplement par une force de ressort (27),
**CARACTERISEE EN CE QUE**,
la valeur du couple de rotation est variable manuellement en continu ou par paliers au moyen d'un dispositif de réglage (15) muni d'un élément de réglage (31) accessible depuis l'extérieur et la tension de la force de ressort est variable avec le dispositif de réglage (15).

2. Pièce à main (3) pour des applications médicales, en particulier pour un dispositif de traitement médical ou dentaire, de préférence pour la préparation d'un canal de racine de dent ou pour l'exercice d'une commande de mouvement ou d'entraînement en rotation avec
- un porte-outil (5) disposé dans la zone avant de la pièce à main (3),
- un accouplement de retenue (16) pour la fixation amovible de l'outil (6) dans le porte-outil (5)
- et un raccordement d'entraînement (8) s'étendant vers l'avant dans la pièce à main (3) pour un entraînement de rotation et/ou de levée du porte-outil (5),
**CARACTERISEE EN CE QUE**,
dans la zone arrière (2) de la pièce à main (3) sont agencés un dispositif de logement (55) pour une batterie (56), un dispositif de commande électronique (57) et un moteur d'entraînement électrique (7),
**en ce que** le dispositif de commande électronique (57) limite le couple de rotation ou d'entraînement transmissible à une valeur de couple d'entraînement de mouvement ou de rotation maximum,
et **en ce que** la valeur du couple de rotation est variable manuellement en continu ou par paliers au moyen d'un dispositif de réglage avec un élément de réglage (31) accessible depuis l'extérieur.

3. Pièce à main (3) pour des applications médicales, en particulier pour un dispositif de traitement médical ou dentaire, de préférence pour une préparation de canal d'une racine dentaire ou pour l'exercice d'une commande de mouvement ou d'entraînement en rotation avec
- un porte-outil (5) disposé dans la zone d'extrémité avant de la pièce à main (3),
- un accouplement de retenue (16) pour la fixation amovible de l'outil (6) dans le porte-outil (5),
- un raccord d'entraînement (8) s'étendant vers l'avant dans la pièce à main (3) pour un entraînement de rotation et/ou de levée du porte-outil (5),
- et un accouplement de surcharge (14) disposé dans le raccord d'entraînement (8), qui limite le couple de rotation ou d'entraînement de mouvement transmissible à une valeur de couple de rotation ou d'entraînement de mouvement maximum,
**CARACTERISEE EN CE QUE**,
- dans la zone d'extrémité arrière (2) de la pièce à main (3) sont agencés un dispositif de logement (55) pour une batterie (56), un dispositif de commande électronique (57) et un moteur d'entraînement électrique (7),
et **en ce que** la valeur du couple de rotation est variable manuellement en continu ou par paliers au moyen d'un dispositif de réglage (15) avec un élément de réglage (31) accessible depuis l'extérieur.

4. Pièce à main selon la revendication 1 ou 3,
**CARACTERISEE EN CE QUE**,
la pièce à main (3) est raccordée par un accouplement rapide (4) amovible avec une partie de connexion arrière (2,Fig. 1 et 2).

5. Pièce à main selon la revendication 2,
**CARACTERISEE EN CE QUE**,
la pièce à main (3) est raccordée au moyen d'un accouplement rapide (4) de façon amovible à une zone d'extrémité arrière (2, fig.13) comportant le dispositif de logement (55), un dispositif de commande électronique (57) et un moteur d'entraînement électronique (7).

6. Pièce à main selon la revendication 1,
**CARACTERISEE EN CE QUE**,
l'accouplement de surcharge (14) est disposé dans une section de raccordement d'entraînement (10,11,11a,12a), s'étendant jusqu'au porte-outil (5), du raccordement d'entraînement (8).

7. Pièce à main selon l'une des revendications précédentes 1,3 et 4,
**CARACTERISEE EN CE QUE**,
l'accouplement de surcharge (14) est disposé dans la zone médiane ou arrière de la pièce à main oblongue (3).

8. Pièce à main selon l'une des revendications précédentes,
**CARACTERISEE EN CE QUE**,
il est prévu un engrenage (13) de préférence réducteur, qui est agencé dans la pièce à main (3), en particulier dans sa zone médiane ou arrière, ou dans la partie de connexion (2).

9. Pièce à main selon la revendication 8,
**CARACTERISEE EN CE QUE,**
l'accouplement de surcharge (14) est monté en amont de l'engrenage (13) sur son côté dirigé vers le porte-outil (5).

10. Pièce à main selon l'une des revendications 1,3 et 4 ainsi que 6 à 9,
**CARACTERISEE EN CE QUE**,
l'accouplement de surcharge (14) présente deux parties d'accouplement (14a,14b) montées de façon rotative, qui sont agencées coaxialement entre elles et présentent respectivement une face de friction (14c,14d) symétrique en rotation, sur lesquelles sont juxtaposées les parties d'accouplement (14a,14b) avec une précontrainte par effet ressort élastique.

11. Pièce à main selon la revendication 10,
**CARACTERISEE EN CE QUE**,
les faces de friction (14c,14d) s'étendent dans un plan à angle droit par rapport à l'axe médian longitudinal (21) des parties d'accouplement (14a,14b).

12. Pièce à main selon l'une des revendications précédentes 3 et 4 ainsi que 6 à 11,
**CARACTERISEE EN CE QUE**,
une partie d'accouplement (14a) est montée de façon déplaçable axialement et est précontrainte par une force de ressort (27) dans sa position d'accouplement.

13. Pièce à main selon l'une des revendications précédentes 1,3 et 4 ainsi que 6 à 12,
**CARACTERISEE EN CE QUE**,
l'accouplement de surcharge (14) présente deux parties d'accouplement (14a,14b) montés de façon rotative, qui sont agencées coaxialement l'une par rapport à l'autre et comportent sur leurs côtés en regard l'un de l'autre dans la direction périphérique, des dépressions (14f) et des élévations (14c) s'alternant et s'adaptant l'une à l'autre avec des flancs axialement obliques et arrondis, tandis qu'une ou les deux parties d'accouplement (14a) est ou sont montée(s) de façon élastique et souple en vue du désaccouplement.

14. Pièce à main selon l'une des revendications précédentes 1,3 et 4 ainsi que 6 à 13,
**CARACTERISEE EN CE QUE**,
l'accouplement de surcharge (14) présente deux parties d'accouplement (14a,14b), qui sont agencées coaxialement l'une par rapport à l'autre et sur leurs côtés en regard l'un de l'autre dans la direction périphérique, comportent des dépressions (14f) successives et opposées avec des flancs axialement obliques et arrondis, tandis que l'une ou les deux parties d'accouplement (14a) est ou sont montée(s) de façon élastique et souple en vue du désaccouplement et tandis qu'entre les parties d'accouplement (14a,14b) sont montés des corps de roulement (52), en particulier des billes dont la dimension en section transversale est supérieure à la somme des profondeurs des dépressions (14f).

15. Pièce à main selon la revendication 13 ou 14,
**CARACTERISEE EN CE QUE**,
l'angle (W1) inscrit par les flancs est obtus et de préférence, il est d'environ 135 à 170°.

16. Pièce à main selon la revendication 14 ou 15,
**CARACTERISEE EN CE QUE**,
les dépressions (14f) sont formées de préférence par des calottes (54) adaptées à la forme et à la dimension des corps de roulement (52), en particulier en forme de section de sphère.

17. Pièce à main selon l'une des revendications précédentes,
**CARACTERISEE EN CE QUE**,
l'élément de réglage (31) est réglable dans la direction périphérique et/ou axialement et de préférence, sur la périphérie de la pièce à main (3).

18. Pièce à main selon l'une des revendications précédentes,
**CARACTERISEE EN CE QUE**,
un dispositif de blocage (F) pour l'enclenchement amovible dans la position respectivement réglée est affecté au dispositif de réglage (15) ou à l'élément de réglage (31).

19. Pièce à main selon la revendication 18,
**CARACTERISEE EN CE QUE**,
le dispositif de blocage (F) est formé par un dispositif d'enclenchement qui, de préférence, est manuellement actionnable pour son effacement.

20. Pièce à main selon l'une des revendications 12 à 19,
**CARACTERISEE EN CE QUE**,
la précontrainte de la force de ressort est variable avec le dispositif de réglage (15).

21. Pièce à main selon l'une des revendications précédentes,
**CARACTERISEE EN CE QUE**,
l'élément de réglage (31), de préférence formé par un poussoir ou un anneau rotatif, est raccordé au dispositif de réglage (15) par l'intermédiaire d'un élément de raccordement radial (32).

22. Pièce à main selon l'une des revendications précédentes,
**CARACTERISEE EN CE QUE**,
le dispositif de réglage (15) présente un mécanisme à came (48) ou un mécanisme fileté se trouvant en liaison d'entraînement avec l'anneau rotatif (31a).

23. Pièce à main selon l'une des revendications précédentes,
**CARACTERISEE EN CE QUE**,
sur la course de réglage de l'élément de réglage (31) est ou sont agencés une échelle (36a,36b) et/ou un marquage (36c) respectivement avec une contre-échelle ou avec un contre-marquage ou avec un index et/ou une ou plusieurs positions de crans (51a,51b,51c) sur l'élément de réglage (31) et avec une partie limitrophe, en particulier la partie porteuse de l'élément de réglage (31).

24. Pièce à main selon la revendication 23,
**CARACTERISEE EN CE QUE**,
le marquage (36c) présente différents signes ou couleurs.

25. Pièce à main selon l'une des revendications précédentes,
**CARACTERISEE EN CE QUE**,
il est prévu plusieurs outils différents (6), qui se distinguent entre eux de par leur forme et/ou dimension et/ou solidité.

26. Pièce à main selon la revendication 25,
**CARACTERISEE EN CE QUE**,
les outils (6) présentent respectivement sur le fût d'outil (6a) un marquage (36c), tandis que la position du marquage (36c) et/ou des positions de cran (51a,51b,51c) sur la course de réglage correspond à une valeur de couple de rotation ou d'entraînement déterminé, par exemple à une valeur maximum de couple de rotation ou d'entraînement de l'outil concerné (6).
